(19) **Europäisches Patentamt European Patent Office Office européen des brevets**

(11) **EP 2 229 949 B1**

(12) **EUROPEAN PATENT SPECIFICATION**

(45) Date of publication and mention of the grant of the patent:
**21.12.2011 Bulletin 2011/51**

(51) Int Cl.:
*A61K 35/74* (2006.01)    *C12N 1/20* (2006.01)
*C12Q 1/04* (2006.01)    *A61K 8/99* (2006.01)
*A61K 9/00* (2006.01)    *A61P 1/02* (2006.01)

(21) Application number: **10168703.6**

(22) Date of filing: **14.06.2005**

(54) **USE OF LACTIC ACID BACTERIA FOR DECREASING GUM BLEEDING AND REDUCING ORAL INFLAMMATION**

VERWENDUNG VON MILCHSÄUREBAKTERIEN ZUR VERRINGERUNG VON ZAHNFLEISCHBLUTEN UND MUNDENTZÜNDUNG

UTILISATION DE BACTERIES D'ACIDE LACTIQUE POUR REDUIRE LE SAIGNEMENT DES GENCIVES ET L'INFLAMMATION ORALE

(84) Designated Contracting States:
**AT BE BG CH CY CZ DE DK EE ES FI FR GB GR HU IE IS IT LI LT LU MC NL PL PT RO SE SI SK TR**

(30) Priority: **14.06.2004 US 580279 P**
     **08.06.2005 US 147880**

(43) Date of publication of application:
**22.09.2010 Bulletin 2010/38**

(62) Document number(s) of the earlier application(s) in accordance with Art. 76 EPC:
**05752662.6 / 1 765 282**

(73) Proprietor: **Biogaia AB**
**112 27 Stockholm (SE)**

(72) Inventor: **Möllstam, Bo**
**44321, LERUM (SE)**

(74) Representative: **Fagerlin, Heléne**
**Albihns.Zacco**
**Valhallavägen 117**
**Box 5581**
**114 85 Stockholm (SE)**

(56) References cited:
**EP-A1- 1 312 667**    **EP-A1- 1 498 039**
**WO-A2-99/47657**    **US-A- 3 992 519**
**US-A1- 2003 077 814**    **US-A1- 2004 146 493**

- **VALEUR N ET AL: "Colonization and Immunomodulation by Lactobacillus Reuteri ATCC 55730 in the Human Gastrointestinal Tract" APPLIED AND ENVIRONMENTAL MICROBIOLOGY, AMERICAN SOCIETY FOR MICROBIOLOGY, US LNKD- DOI:10.1128/AEM. 70.2.1176-1181.2004, vol. 70, no. 2, 1 February 2004 (2004-02-01), pages 1176-1181, XP002992069 ISSN: 0099-2240**
- **"BioGaia's Healthy bacterium reduces the risk for tooth decay BioGaia AB's patented lactic acid bacterium, Lactobacillus reuteri, inhibits growth of the cariogenic bacterium Streptococcus mutans" BIOGAIA PRESS RELEASE, XX, XX, 22 October 2002 (2002-10-22), page 1, XP002980249**
- **DATABASE BIOSIS [Online] BIOSCIENCES INFORMATION SERVICE, PHILADELPHIA, PA, US; 19 October 2001 (2001-10-19), JONSSON H ET AL: "Addition of mucin to the growth medium triggers mucus-binding activity in different strains of lactobacillus reuteri in vitro" XP002980250 retrieved from BIOSIS**

Remarks:
The file contains technical information submitted after the application was filed and not included in this specification

**Description**

BACKGROUND OF THE INVENTION

Field of the Invention

This invention relates to the selection and use of nonpathogenic, anti-inflammatory and anti-bleeding lactic acid bacteria strains, and products and methods using such strains for treatment and prophylaxis of bleeding gum and gingivitis.

Description of the Related Art

Bleeding from the gums has been considered to be mainly due to inadequate plaque removal from the teeth at the gum line. Plaque is a sticky material that develops on the exposed portions of the teeth, consisting of bacteria, mucus, and food debris. It is a major cause of tooth decay. Plaque that is not removed mineralizes into a hard deposit called tartar that becomes trapped at the base of the tooth. Plaque and tartar irritate and inflame the gingiva. Ultimately, this will lead to increased bleeding and a more advanced form of gum and jawbone disease known as periodontitis.

Gingivitis is one of the most commonly occurring chronic inflammations in humans. Gingivitis, a form of periodontal disease, is a condition when the gingiva has lost its normal appearance and has become swollen, soft and red.

Common causes of gum and tooth problems, such as gingivitis, also include periodontitis (advanced form of gingivitis), anticoagulants such as Coumadin (warfarin) and heparin, toothbrush abrasion, improper flossing, infection, which can be either tooth or gum related, Vitamin C deficiency, Vitamin K deficiency, hormonal changes during pregnancy, chemical irritants such as aspirin, leukemia, placement of new dentures leading to denture sores/irritations, and idiopathic thrombocytopenic purpura. Periodontal disease, generally, is when inflammation and infection destroy the tissues that support the teeth, including the gingiva (gums), the periodontal ligaments, and the tooth sockets (alveolar bone). Misaligned teeth, rough edges of fillings, and ill fitting or unclean mouth appliances (such as orthodontic appliances, dentures, bridges, and crowns) can irritate the gums and increase the risk of gingivitis. Medications such as phenytoin and birth control pills, and ingestion of heavy metals such as lead and bismuth are also associated with gingivitis.

Gingivitis and periodontitis are caused by several mechanisms including accumulation of bacteria in the tooth pocket starting an inflammatory reaction and the long-term effects of plaque deposits. If the inflammation and degradation of collagen increases and reaches further down into the pocket the gingivitis develops into periodontitis. For the patient the immediate consequence of the sore and bleeding gum is that tooth-cleaning becomes difficult. In acute and severe cases the patient may be more generally affected and fever may occur. Further, side effects of oral inflammation and bleeding have been reported to be associated with both heart disease and spontaneous pre-term birth. Complications include the recurrence of gingivitis, periodontitis, infection or abscess of the gingiva or the jaw bones, and trench mouth.

Since gingivitis is the first phase leading to periodontitis, treatment and preventative measures are among the more common challenges for today's dentists. Today the first measure for treating gingivitis is to improve the patient's oral hygiene and sometimes treatment with chlorhexidine or antibiotics is used.

If a dentist determines that the patient has some bone loss or that the gums have receded from the teeth, the standard treatment is an intensive deep-cleaning, non-surgical method called scaling and root planning (SRP). Scaling scrapes the plaque and tartar from above and below the gum line. Root planning smoothes rough spots on the tooth root where germs collect and helps remove bacteria that can contribute to the disease. This smooth, clean surface helps allow the gums to reattach to the teeth.

A relatively new drug in the arsenal against serious gum disease called Periostat (doxycycline hyclate) was approved by the FDA in 1998 to be used in combination with SRP. While SRP primarily eliminates bacteria, Periostat, which is taken orally, suppresses the action of collagenase, an enzyme that causes destruction of the teeth and gums. Antibiotic treatments can be used either in combination with surgery and other therapies, or alone, to reduce or temporarily eliminate the bacteria associated with periodontal disease. However, doctors, dentists and public health officials are becoming more concerned that overuse of these antibiotics can increase the risk of bacterial resistance to these drugs. When germs become resistant to antibiotics, the drugs lose the ability to fight infection. There are also antibiotic gels, fibers or chips applied directly to the infected pocket. In some cases, a dentist will prescribe a special anti-germ mouth rinse containing chlorhexidine to help control plaque and gingivitis. Also available is over-the-counter toothpaste containing the antibacterial triclosan. The antibacterial ingredient is claimed to reduces plaque and resulting gingivitis but clinical effects are weak.

It is known that several different bacterial species (among others *Actinobacillus actinomycetemcomitans, Porphyromonas gingivalis , Bacterioides forsythus, Campylobacter rectus,* and *Selenomonas noxia*) are implicated in the pathogenesis of periodontic diseases. It is not known if these bacteria are causing the diseases or if their presence occur as an opportunistic result of the change of the composition of the biofilms which in turn causes the disease to progress.

The disease progression is dependent on several changes in the microbiota, on the gingival crevicular fluid and on the interaction with the innate host defense.

Periodontal diseases are very wide-spread in the industrialized world. Many people experience gingivitis to a varying degree. It usually develops during puberty or early adulthood due to hormonal changes and may persist or recur frequently, depending on how healthy the teeth and gums are. Depending on age and gender, 45-70% of all US citizens above age 13 are affected by gingival bleeding. The prevalence is highest among those who are 13-17 years old, and lowest at 35-44 years of age, after which the prevalence slightly goes up again. When it comes to the most severe form of periodontal disease, periodontitis (defined as attachment loss exceeding 3 mm) occurs in 30-40% of people 30-39 years of age and then increases linearly with increasing age to 85-90% at 80-90 years of age. The situation is probably similar in Europe and the rest of the industrialized world. Swedish data indicate that close to 3 million inhabitants have problems with bleeding gums.

Normally a dentist is consulted if signs of gingivitis are present. The dentists will examine the mouth and teeth and look for soft, swollen, red-purple gingiva. Deposits of plaque and tartar may be visible at the base of the teeth. The gums are usually painless or mildly tender. No further testing is usually done, although dental x-rays and dental gingival probing (measuring the amount of bone) may be performed to determine whether periodontitis (spread of inflammation to the supporting structures of the teeth) has developed. The removal of plaque from inflamed gums may be uncomfortable. Over-the-counter anti-inflammatory medications will sometimes be used to ease any discomfort from a rigorous cleaning. Healthy gums are pink and firm in appearance. Strict oral hygiene is recommended to be maintained for the patient's whole life or gingivitis will recur.

The goal of gingivitis treatment is to reduce the gingival inflammation and bleeding. Normal treatment includes that the teeth are cleaned thoroughly by the dentist or dental hygienist. This may involve using various instruments or devices to loosen and remove deposits from the teeth (scaling). The dentist or hygienist will often demonstrate brushing and flossing techniques. Professional tooth cleaning in addition to brushing and flossing may be recommended twice per year or more frequently for severe cases. Antibacterial mouth rinses or other aids may be recommended in addition to frequent, careful, tooth-brushing and flossing.

For periodontitis treatment, the primary strategy is similar to the treatment of gingivitis; however, due to the severity of the disease, additional procedures may be necessary. The goal of treatment is to reduce inflammation, eliminate pockets if present, and address any underlying causes. Dental irritants, such as rough surfaces of teeth or dental appliances, should be repaired. It is important to have the teeth cleaned thoroughly. Therefore, scaling is strongly recommended. Meticulous home oral hygiene is necessary after professional tooth cleaning to limit further destruction. The dentist or hygienist will demonstrate brushing and flossing techniques. With periodontitis, professional tooth cleaning is often recommended more frequently than the standard twice a year. Surgical treatment may be necessary. Deep pockets may need to be opened and cleaned. Loose teeth may need to be supported. Extraction (removal) of a tooth may be necessary for advanced periodontitis so destruction does not spread to adjacent teeth.

In spite of the relative success of the acute treatment performed by professionals, it is well known that many diagnosed and treated patients will come back at the next appointment with a similar or worse condition. Regular professional tooth cleaning is important to remove plaque that may develop even with careful brushing and flossing. Many dentists recommend having the teeth professionally cleaned at least every 6 months.

In general the possibility of effective antibacterial activity by several lactobacilli is well known, but not much has been known about differences between lactic acid bacteria strains in their ability to reduce host inflammation, nor that such strains could be selected, however, this is now possible.(WO2004031368)

The oral cavity of humans and other mammals contains many different species of bacteria, including a number of different species of lactic acid bacteria. There has been some speculation that that lactic acid bacteria can positively affect oral inflammation and be of benefit in terms of reduced gingivitis and gum bleeding. For example, *Lactobacillus reuteri* is a major component of the lactobacilli population that naturally inhabits humans and animals. The organism has been extensively studied as a probiotic over the last ten years and found to possess a number of interesting properties. The invention described herein is different from the general probiotic use of lactic acid bacteria in that the bacteria need not be ingested; the presence of the lactic acid bacteria locally on the oral bio-film close to the gingival area is sufficient for the anti-bleeding effects of the invention. To use the strains of the invention a person can just use a chewing gum or a mouth-rinse product that has the lactic acid bacteria in it and spit out the product after sufficient time at this locale. The anti-bleeding and anti-inflammatory effect can be detected within days.

Strains of a wide variety of *Lactobacillus* species, including *Lactobacillus reuteri,* have been used in anti-microbial formulations. *Lactobacillus reuteri* is one of the naturally occurring inhabitants of the gastrointestinal tract of animals, and is routinely found in the intestines, and occasionally in the birth channel, breast milk and mouth of healthy animals, including humans. It is known to have antibacterial activity. See, for example, U.S. Patent Nos. 5,439,678, 5,458,875, 5,534,253, 5,837,238, and 5,849,289. When *L. reuteri* cells are grown under anaerobic conditions in the presence of glycerol, they produce the antimicrobial substance known as reuterin (ß-hydroxy-propionaldehyde). Other antimicrobial substances beside the traditional organic acids have also been reported such as "Reutericyclin" (Höltzel, A. et al. Angewandte Chemie International Edition 39, 2766 - 2768, 2000) and "PCA (pyroglutamic acid)" (Yang, Z. Dissertation, Univ. of Helsinki, March 2000), and "Reutericin 6" (Toba T, et al., Lett Appl Microbiol 13: 281-6.). Lactobacilli, including *L. reuteri,* are also well known to have the ability to inhibit various pathogenic organisms through local competition of nutrients and other metabolic interactions.

Mucin binding proteins of *L. reuteri* have been isolated and described. See, for example, U.S. Patent No. 6,100,388. *Lactobacillus* strains have been reported to adhere to various cell lines and host mucus (Klemm, P. and Schembri, M. A. (2000) Bacterial adhesins: function and structure. Int. J. Med. Microbiol. 290, 27-35. ) It has however not been so well known that there are important differences between a *Lactobacillus* strains ability to adhere to oral mucin and mucin from other sources. Some strains are good at adhering to both oral mucin and other mucin, for example, gastric mucin, others are only good at adhering to gastric mucin but less good to oral mucin, others does not adhere well to any kind of mucin. It is therefore a part of the selection method of this invention to use oral mucin to find the best strains.

While the possibility of effective anti-bacterial, anti-inflammatory, and binding characteristics by *L. reuteri* and some other lactic acid bacteria is known, as well as some bacteria's ability to secrete vitamin K (menaquinones), it was not previously known that substantial differences existed between lactic acid bacteria strains in their ability to decrease gum bleeding and reduce gingivitis, nor that such strains could be selected. It is now also generally recognized that menaquinones biosynthesis is increased in anaerobiosis (Bentley et. al., Microbiological review Sept. 1982, p.241-280) meaning that the selected strains ability to produce vitamin K to help decrease the bleeding, will increase in areas closest to the gingival, where it is best needed.

Vitamin K, is a group of three related substances. K1-Phytonadione - from plants, K2-Menaquinone - from bacteria, K3-Menadione - synthetic. Vitamin K is necessary for normal blood clotting. It is required for the synthesis of prothrombin and other proteins (Factors IX, VII, and X) involved in blood coagulation.

Several cases of excessive bleeding have been reported in people who take antibiotics. (Huilgol VR, et al. Am J Gastroenterol 1997;92:706-7) This side effect may be the result of reduced vitamin K activity and/or reduced vitamin K production by bacteria. One study showed that people who had taken broad-spectrum antibiotics had lower liver concentrations of vitamin K2 (menaquinone), though vitamin K1 (phylloquinone) levels remained normal. Several antibiotics appear to exert a strong effect on vitamin K activity, while others may not have any effect. Moreover, most multivitamins do not contain vitamin K.

It has unexpectedly been found that *Lactobacillus* strains specifically selected according to the present invention for their anti-microbial, anti-inflammatory, oral mucin (teeth-biofilm) adhesion properties and their ability to produce vitamin K (menaquinones) are better in decreasing gum bleeding and reducing gingivitis.

It is therefore an object of the invention to provide better strains of lactic acid bacteria which have been selected for their capability to decrease gum bleeding and reduce gingivitis in the mouth through antimicrobial activity, anti-inflammatory activity, such as that due to *Porphyromonas gingivalis,* good capability of adhering to oral mucin in combination with good secretion of vitamin K and thereby successfully prevent, reduce or treat gum bleeding and gingivitis. It is a further object of the invention to provide products containing said strains for administration to humans. Such products could also be used to decrease inflammation and bleeding on skin and other local surfaces of the body.

It is also an object of the invention to provide strains of lactic acid bacteria which have been selected for their capability of reducing oral inflammation, such as that due to *Porphyromonas gingivalis* and also such strains that are good producers of vitamin K to decrease gum bleeding.. It is a further object of the invention to provide products containing said strains, including agents for treatment or prophylaxis of bleeding gum and gingivitis for administration to humans.

Other objects and advantages will be more fully apparent from the following disclosure and appended claims.

SUMMARY OF THE INVENTION

The invention herein relates to the use of nonpathogenic, anti-inflammatory and anti-bleeding lactic acid bacteria strains, and products and methods using such strains for treatment and prophylaxis of bleeding gum and gingivitis caused by oral inflammation.

Other objects and features of the inventions will be more fully apparent from the following disclosure and appended claims.

BRIEF DESCRIPTION OF THE DRAWINGS

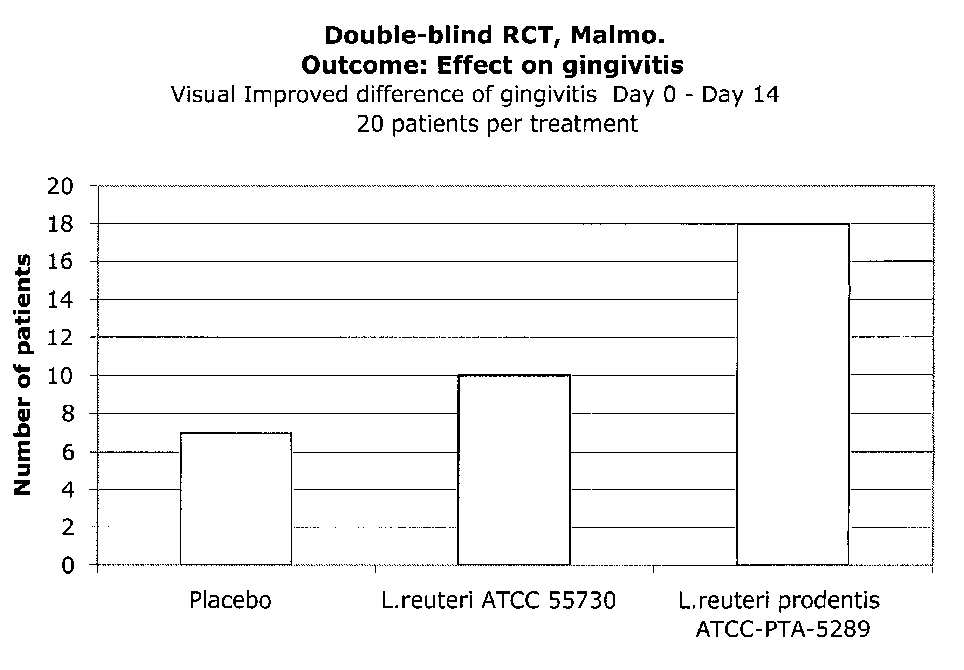

Figure 1 is a bar graph showing the effect of lactic acid bacteria on visual improved effect on bleeding gum/gingivitis. L. r. prodentis vs. placebo: $p < 0.005$, L. r. prodentis vs. L.r.ATCC55730: $p < 0.05$, L. r. ATCC55730 vs. placebo: n.s. (Fisher's exact)

DETAILED DESCRIPTION OF THE INVENTION AND PREFERRED EMBODIMENTS THEREOF

The invention herein relates to the use of nonpathogenic, anti-inflammatory and anti-bleeding lactic acid bacteria strains, and products and methods using such strains for treatment and prophylaxis of bleeding gum and gingivitis caused by oral inflammation.

The present invention provides a product, for decreasing gum bleeding and reducing gingivitis, utilizing selected strains in such products, especially selected from *Lactobacillus reuteri* such as *L. reuteri* "Prodentis" (ATCC PTA-5289) and L. *reuteri* FJ3 (ATCC PTA-5290). These strains are available to the public at the American Type Culture Collection (Rockville, MD) having been deposited there on July 29, 2003, and further *L. reuteri* (ATCC 55730) deposited December 18, 1995. The deposits of *Lactobacillus reuteri* "Prodentis" (ATCC PTA-5289), *L. reuteri* FJ3 (ATCC PTA-5290) and *L. reuteri* (ATCC 55730) meet the requirements of the Budapest Treaty.

In the selection method used herein, the inhibiting effects of bacterial species (among others *Actinobacillus actinomycetemcomitans*, *Porphyromonas gingivalis*, *Bacterioides forsythus*, *Campylobacter rectus*, and *Selenomonas noxia*) implicated in the pathogenesis of periodontic diseases are examined by traditional microbiological methods using the bacterial cells. The adhesion capabilities are measured using oral mucin coated in microtiter wells (ref. Jonsson et al. 2001 FEMS Microbiol.lett. 204: 19-22).

Selection of vitamin K producing lactic acid bacteria is done by standard methods such as HPLC analysis (ref Conly JM, Stein K. Am J Gastroenterol. 1992 Mar;87(3):311-Quantitative and qualitative measurements of K vitamins) of the test bacteria anaerobically grown in MRS media, or by a genetic probe analyzing the test bacteria. As known by a person in the art, all growth of strains and analysis for vitamin K should be done under yellow light as the substance is sensitive to photooxidation.

The product of the invention can be any product for placement in the mouth as a local preventative or treatment for gum bleeding and gingivitis, also such products as mouthwashes or other specified health products, chewing gum, lozenges and the like.

The concentration of selected *Lactobacillus* cells needed for effectiveness of a product of the invention depends on the type of formulation to be used (or the time of use in the mouth), but it is usually preferable to have equivalent of about $10^5$-$10^8$ CFU (colony-forming units) or more per daily oral placement of a product. Amounts up to about $10^{10}$-$10^{11}$ CFU are possible and can be used to increase efficacy without adversely affecting the product's organoleptic characteristics (its flavor or smell).

Preferably the product of the invention does not contain other antibacterial components, at least none that inhibit or kill selected lactic acid bacterial strain(s) or interfere with the anti-inflammatory or anti-bleeding activity.

The product of the invention may however comprise any other ingredients that is active against gingivitis and does not interfere with the Lactobacillus according to the invention. The product may also comprise excipients and additives known in the art. The expression "comprising" as used herein should be understood to include, but not be limited to, the stated items.

The strain(s) of lactic acid bacteria can be an additive mixed into the ingredients or kneaded into or coated on the product by means known in the art for formulation of products of that type. When using cells and if preparation of the selected food or other product of the invention requires a heating step, the lactic acid bacterial strain(s) should be added after the heating. Once the selected lactic acid bacteria cells are in the product, it is preferred not to heat the product to 60-70 degrees C or above for a longer period of time.

The features of the present invention will be more clearly understood by reference to the following examples, which are not to be construed as limiting the invention.

Further disclosed are the following points:

1. A method for selecting a lactic acid bacterial strain for external administration to a human, comprising selecting a strain of *Lactobacillus* having anti-microbial, anti-inflammatory and oral mucin adhesion properties and having the ability to produce vitamin K.

2. A method of decreasing gum bleeding and reducing gingivitis in a person's mouth, comprising administering to the person cells of the lactic acid bacterial strain selected by the method of point 1.

3. The method of point 2, wherein $10^5$- $10^8$ colony-forming units of the lactic acid bacterial strain are administered daily to the person

4. The method of point 2, wherein the cells are administered orally to the person.

5. The method of point 4, wherein $10^5$-$10^8$ colony-forming units of the lactic acid bacterial strain are administered daily to the person.

6. A product containing cells of a lactic acid bacterial strain selected by the method of point 1 for external administration to a human.

7. The product of point 6, further comprising agents for treatment or prophylaxis of bleeding gum and gingivitis.

8. The product of point 7, wherein the product is formulated as a product selected from the group consisting of mouthwashes, chewing gum, and lozenges.

9. The product of point 6, wherein the lactic acid bacterial strain is selected from the group consisting of *Lactobacillus reuteri* "Prodentis" (ATCC PTA-5289), *L. reuteri* FJ3 (ATCC PTA-5290) and *L. reuteri* (ATCC PTA 55730).

10. A strain of lactic acid bacteria selected by the method of point 1.

11. Use of a strain of *Lactobacillus* having anti-microbial, anti-inflammatory and oral mucin adhesion properties and having the ability to produce vitamin K for the production of a pharmaceutical for decreasing gum bleeding and reducing gingivitis in a person's mouth.

Example 1. Method of Selection of Strains

The selection of the lactic acid bacteria strains to be used according to this invention can be done in the following four step manner:

a) Evaluation of inhibiting effect of *Porphyromonas gingivalis* by cells of lactic acid bacteria strains

An example of a strain to use to measure the inhibitory effect is *Porphyromonas gingivalis,* ATCC33277 (available from The American Type Culture Collection, Manassas, VA, USA). The isolate is grown in trypticase soy broth (Difco, Detroit, USA) supplemented with 0.5 % yeast extract (Difco) (TSBY). The cells are harvested during the exponential growth phase by centrifugation at 1000 x g, washed twice with PBS and resuspended in the same buffer. The cell suspensions are subjected to a low-intensity ultrasonic device to disperse bacterial aggregates.

The test lactic acid bacteria strain is grown in MRS broth (Difco), and harvested during the exponential growth phase by centrifugation at 1000 x g, washed twice with phosphate buffered saline (PBS; pH 6.8) and re-suspended in the same buffer.

The optical densities of the bacterial suspensions are measured in a 1.0 ml cuvette with a 1 cm light path, and the suspensions are adjusted to a final concentration of $1.0 \times 10^8$ CFU (colony forming unit)/ml.

The inhibitory assay is conducted as follows: the suspension of *P. gingivalis* and the suspension of lactic acid bacteria are mixed in the ratios of 100-0, 75-25, 50-50 and 25-75 in sterile centrifugation tube (total volume 100 μL), the BHI broth, up to 10 ml, is added, vortex mixed for ten seconds, and incubated for 90 min at 37°C with gentle shaking. As a control, the suspension of *P. gingivalis* is mixed with an equal volume of PBS in the control tubes (free of lactic acid bacteria). Afterwards each suspension is washed by centrifugation at 1000 x g, washed twice with PBS, and plated on MS agar to determine the CFU count of *P. gingivalis*. The % survival of *P. gingivalis* is obtained from following formula.

$$\% \text{ survival of } P.\ gingivalis = \frac{\text{CFU of } P.\ gingivalis \text{ incub. w lactic acid bacteria } \times 100}{\text{CFU of } P.\ gingivalis \text{ incubated with PBS}}$$

The assay should be carried out with minimum triplicate samples. All the numerical data obtained should be statistically analyzed.

In this example using the above method *L. reuteri* "Prodentis" (ATCC PTA-5289) *L. reuteri* FJ3 (ATCC PTA-5290) and *L. reuteri* (ATCC 55730) are selected.

b) Evaluation of adhesion capabilities to oral mucin / oral biofilm of lactic acid bacteria strains

The lactic acid bacteria strains to be tested are collected. The bacteria are grown at 37°C in MRS broth (Difco) for 16h. Plates are incubated in anaerobic jars under $CO_2+N_2$ atmosphere (GasPak System, BBL, Becton Dickinson Microbiology Systems, Cockeysville, MD, USA).

Oral mucus as human saliva are collected, centrifuged, sterile filtered and coated into microtiter wells as described. The mucus are collected in 200 ml ice-cold phosphate-buffered saline (PBS) (8.0 g NaCl, 0.2 g KCl, 1.44 g $Na_2HPO_42H_2O$ and 0.2 g $KH_2PO_4$ per 1000 ml of $dH_2O$) and supplemented with 0.05% Tween 20 (PBST). The resulting suspension is centrifuged first at 11000g for 10 min and then at 26000g for 15 min in order to remove cells and particulate matter. As an alternative mucin is gastric mucin (Sigma, M1778) used. The crude mucus preparation is stored at 20 °C. The mucus material is diluted to approximately 100 $\mu$g ml-1 in 50 mM Na2CO3 buffer, pH 9,7 and incubated overnight in microtiter wells (Greiner) (150 $\mu$l per well) at 4 °C with slow rotation. The wells are blocked with PBS with 1% Tween 20 for 1h and thereafter washed with PBST. Wells coated with BSA are used as controls.

The strains to be tested are grown as per above, washed once in phosphate-buffered saline (PBS) (pH 7.3) supplemented with 0.05 %Tween 20 (PBST) and diluted to $OD_{600}$ 0.5 in the same buffer. One hundred microliters bacterial suspension is added to each well and incubated over night at 4°C. The wells are washed 4 times with PBST and binding examined with an inverted microscope. The buffer is poured off and, after the wells had dried, the binding is measured over the whole surface of the well in an BioRad Gel Doc 2000 instrument (BioRad Laboratories, Herkules, CA, USA). All measurements are done in triplicate.

In using the above method in this example *L. reuteri* "Prodentis" (ATCC PTA-5289) is firstly selected and *L. reuteri* FJ3 (ATCC PTA-5290) and *L. reuteri* (ATCC 55730) selected second alternatives.

c) Evaluation of anti-inflammatory capabilities of lactic acid bacteria strains

Test lactic acid bacteria are grown in de Man, Rogosa, Sharpe (MRS) and Luria-Bertani (LB) media (Difco, Sparks, MD), respectively. Overnight cultures of lactic acid bacteria are diluted to an $OD_{600}$ of 1.0 (representing approximately $10^9$ cells/ml) and further diluted 1:10 and grown for an additional 4, 8 and 24 h. *Porphyromonas gingivalis,* ATCC33277 is cultured for 48 h in Brucella broth (Difco) supplemented with 10% fetal bovine serum (FBS). Cultures are diluted 1:10 and grown for another 24 and 48 h. Bacterial cell-free conditioned medium is collected by centrifugation at 8500 rpm for 10 min at 4°C. Conditioned medium is separated from the cell pellet and then filtered through a 0.22 $\mu$m pore filter unit (Millipore, Bedford, Mass. USA).

Mouse monocyte/macrophage cell lines, RAW 264.7 (ATCC TIB-71) and RAW 264.7 gamma NO(-) (ATCC CRL-2278), are used as a reporter cells for studying the inflammatory response pathway. RAW 264.7 cells are grown in either Dulbecco's Modified Eagle Medium (wild-type) or RPMI Medium 1640 (gamma NO-) (Gibco-Invitrogen, Carlsbad, CA) supplemented with 10% FBS and 2% antibiotic (5000 units/ml Penicillin and 5 mg/ml Streptomycin, Sigma) at 5% $CO_2$ 37°C until 80-90% confluent. Approximately 5 x $10^4$ cells are seeded into 96-well cell culture clusters and allowed to adhere for 2 h prior to lipopolysaccharide (LPS) activation and addition of conditioned medium. Naive RAW 264.7 cells are exposed to purified LPS from *E. coli* serotype 0127:B8 (Sigma). Activation medium is made by adding 2 ng LPS to 20 $\mu$l conditioned medium per well. Macrophages are either pre-incubated or co-incubated with cell-free lactic acid bacteria conditioned medium. Recombinant mIL-10 (R&D Systems, Minneapolis, Min.) is used as a positive control. Cell viability is assessed by Trypan-blue (Invitrogen) exclusion. The presence of TNF-$\alpha$ in cell culture supernatant is measured with a sandwich enzyme immunoassay, Quantikine M® Mouse TNF-$\alpha$ Immunoassay (R & D Systems).

In using the above method *L. reuleri* "Prodentis" (ATCC PTA-5289) *L. reuteri* FJ3 (ATCC PTA-5290) are selected.

d) Evaluation of vitamin K secretion capabilities of lactic acid bacteria strains

Selection of vitamin K producing lactic acid bacteria is done by standard methods such as HPLC analysis (ref Conly JM, Stein K. Am J Gastroenterol. 1992 Mar;87(3):311- Quantitative and qualitative measurements of K vitamins) of the test bacteria anaerobically grown in MRS media, or by a genetic probe analyzing the test bacteria. As known by a person in the art, all growth of strains and analysis for vitamin K should be done under yellow light as the substance is sensitive to photooxidation.

The lactic acid bacteria strains showing best results in both inhibiting of *P. gingivalis* using lactic acid bacteria cells as well as best results in adhesion to oral mucin, anti-inflammatory effect and vitamin K secretion according to the assays above, are selected.

Example 2 Manufacturing of chewing gum products containing selected strain

In this example, *L. reuteri* FJ1 "Prodentis" (ATCC PTA-5289), is selected based on good growth characteristics in general and favorable results in the earlier mentioned selection in Example 1 in order to add the strain to a chewing gum. The *L. reuteri* protectis strain is grown and lyophilized, using standard methods for growing *Lactobacillus* in the industry.

The steps of an example of a manufacturing process of chewing gum containing the selected strain follow, with it being understood that excipients, fillers, flavors, encapsulators, lubricants, anticaking agents, sweeteners and other components of chewing gum products as are known in the art, may be used without affecting the efficacy of the product:

1 Melting. Melt Softisan 154 (SASOL GMBH, Bad Homburg, Germany) in a vessel and heat it to 70 °C to assure complete disruption of the crystalline structure. Then cool it down to 52 - 55 °C (just above its hardening point).

2 Granulation. Transfer *Lactobacillus reuteri* freeze-dried powder to a Diosna high-shear mixer/granulator, or equivalent. Add slowly during approximately 1 minute the melted Softisan 154 to the *Lactobacillus reuteri* powder. No additional massing time is required. Use chopper during the addition.

3 Wet-sieving. Immediately after the granulation, pass the granules through a 1-mm sieving net by using a Tornado mill. The sieved granulate is packed in alupouches ,made out of PVC-coated aluminum foil, sealed with a heatsealer to form a pouch, together with desiccant pouch, and stored refrigerated until mixing. The granulated batch is divided for two tablet batches.

4 Mixing. Mix all the ingredients in a mixer, to a homogenous blend.

5 Compression. Transfer the final blend to the hopper of a rotary tablet press and compress tablets with a total weight of 765 mg, in a Kilian compressor.

6 Bulk packaging. The chewing gums are packed in alu-bags together with a drying pouch of molecular sieve. The alu-pouch is put in a plastic bucket and stored in a cool place at least one week, before final package.

In-process controls, as is standard in the industry, are shown in the following Table 1.

Table 1.

| IPC | Test Method | Limit |
|---|---|---|
| 1 | Appearance Visually | Clear, homogenous solution |
| 2 | Temperature Thermometer | 52 - 55 °C |
| 3 | *L. reuteri* assay | CM003 |
| 4 | Appearance Visually | Cream colored with blue spots, convex tablets plain on both sides. |
| 5. | Uniformity of mass Ph. Eur. | 765 mg $\pm$ 5 % |

In the example herein, the selected *L. reuteri* culture is then added as above at a level of $10^7$ CFU/gram of product, and the chewing gum used by humans as a way to decrease gum bleeding and reducing gingivitis.

As stated above, the product of the invention may be in forms other than chewing gum, for example as a lozenge and other formulations and standard methods of preparing the underling underlying product as are known in the art are beneficially used to prepare the product of the invention including the selected *L. reuteri* culture.

**Claims**

1. A method for selecting a Lactobacillus reuteri strain for use in decreasing gum bleeding and reducing gingivitis in a person's mouth, comprising examining the anti-microbial, anti-inflammatory, the_oral mucin adhesion properties and the ability to produce vitamin K of a lactobacillus reuteri strain; and selecting the strain exhibiting all said capabilities.

2. A product comprising cells of a lactobacillus reuteri strain obtainable by the selection method according to claim 1 to have inhibitory activity against Streptococcus mutans in the mouth, to bind to oral mucins and to have vitamin K producing capabilities for use in decreasing gum bleeding and reducing gingivitis in a person's mouth.

3. The product for use according to claim 2 obtainable by the selection method according to claim 1, wherein $10^5$-$10^8$ colony -forming units of the lactic acid bacterial strain are administered daily to the person.

4. The product for use according to any of claims 2-3, obtainable by the selection method according to claim 1, wherein the cells are administered orally to the person.

5. The product for use according to any of claims 2-4, obtainable by the selection method according to claim 1, further comprising agents for treatment or prophylaxis of bleeding gum and gingivitis.

6. The product for use according to any of claims 2-5, obtainable by the selection method according to claim 1, wherein the product is formulated as a product selected from the group consisting of mouthwashes, chewing gum, and lozenges.

**Patentansprüche**

1. Verfahren zur Auswahl eines Lactobacillus reuteri-Stamms zur Verwendung in der Verringerung von Zahnfleischbluten und Zahnfleischentzündungen im Mund einer Person, welches Prüfen der antimikrobiellen, entzündungshemmenden, und der Oralschleimhaut-Haftungseigenschaften und Prüfen der Fähigkeit zur Herstellung von Vitamin K eines Lactobacillus reuteri-Stamms, sowie auswählen des Stamms umfasst, der alle diese Fähigkeiten aufweist.

2. Produkt, das Zellen eines Lactobacillus reuteri-Stamms umfasst, welcher durch das Auswahlverfahren gemäß Anspruch 1 erhältlich ist, um eine inhibitorische Wirkung gegen Streptokokken-Mutanten im Mund aufzuweisen, um auf die Oralschleimhaut zu binden und um Vitamin K-Herstellungsfähigkeiten aufzuweisen, zur Verwendung in der Verringerung von Zahnfleischbluten und Zahnfleischentzündungen im Mund einer Person.

3. Produkt zur Verwendung gemäß Anspruch 2, das durch das Auswahlverfahren gemäß Anspruch 1 erhältlich ist, worin $10^5$ bis $10^8$ Kolonien-bildende Einheiten des Milchsäurebakterienstamms einer Person täglich verabreicht werden.

4. Produkt zur Verwendung gemäß mindestens einem der Ansprüche 2 bis 3, das durch das Auswahlverfahren gemäß Anspruch 1 erhältlich ist, worin die Zellen der Person oral verabreicht werden.

5. Produkt zur Verwendung gemäß mindestens einem der Ansprüche 2 bis 4, welches durch das Auswahlverfahren gemäß Anspruch 1 erhältlich ist, und weiterhin Mittel zur Behandlung oder Vorbeugung von Zahnfleischbluten und Zahnfleischentzündungen umfasst.

6. Produkt zur Verwendung gemäß mindestens einem der Ansprüche 2 bis 5, welches durch das Auswahlverfahren gemäß Anspruch 1 erhältlich ist, worin das Produkt als ein Produkt formuliert ist, das ausgewählt ist aus Mundwässern, Kaugummi und Lutschtabletten.

**Revendications**

1. Procédé pour sélectionner une souche de Lactobacillus reuteri destinée à être utilisée dans la diminution du saignement des gencives et la réduction de la gingivite dans la bouche d'une personne, comprenant l'examen des propriétés antimicrobiennes, anti-inflammatoires, d'adhésion des mucines buccales et de l'aptitude à produire de la vitamine K d'une souche de Lactobacillus reuteri ; et la sélection de la souche présentant toutes lesdites capacités.

2. Produit comprenant des cellules d'une souche de Lactobacillus reuteri pouvant être obtenue par le procédé de sélection selon la revendication 1 pour avoir une activité inhibitrice contre Streptococcus mutans dans la bouche, pour lier les mucines buccales et pour avoir des capacités de production de vitamine K destiné à être utilisé dans la diminution du saignement des gencives et la réduction de la gingivite dans la bouche d'une personne.

3. Produit destiné à être utilisé selon la revendication 2 pouvant être obtenu par le procédé de sélection selon la revendication 1 où $10^5$ - $10^8$ unités formant des colonies de la souche bactérienne lactique sont administrées quotidiennement à la personne.

4. Produit destiné à être utilisé selon l'une quelconque des revendications 2 - 3 pouvant être obtenu par le procédé de sélection selon la revendication 1 où les cellules sont administrées oralement à la personne.

5. Produit destiné à être utilisé selon l'une quelconque des revendications 2 - 4 pouvant être obtenu par le procédé de sélection selon la revendication 1 comprenant en outre des agents pour le traitement ou la prophylaxie du saignement des gencives et de la gingivite.

6. Produit destiné à être utilisé selon l'une quelconque des revendications 2 - 5 pouvant être obtenu par le procédé de sélection selon la revendication 1 où le produit est formulé sous forme d'un produit choisi dans le groupe consistant en les bains de bouche, une gomme à mâcher et les pastilles.

## Double-blind RCT, Malmo.
## Outcome: Effect on gingivitis
Visual Improved difference of gingivitis  Day 0 - Day 14
20 patients per treatment

EP 2 229 949 B1

## REFERENCES CITED IN THE DESCRIPTION

**Patent documents cited in the description**

- WO 2004031368 A
- US 5439678 A
- US 5458875 A
- US 5534253 A
- US 5837238 A
- US 5849289 A
- US 6100388 A

**Non-patent literature cited in the description**

- **Höltzel, A. et al.** *Angewandte Chemie International Edition,* 2000, vol. 39, 2766-2768
- **Toba T et al.** *Lett Appl Microbiol,* vol. 13, 281-6
- **Klemm, P. ; Schembri, M. A.** Bacterial adhesins: function and structure. *Int. J. Med. Microbiol.,* 2000, vol. 290, 27-35
- **Bentley.** *Microbiological review,* September 1982, 241-280
- **Huilgol VR et al.** *Am J Gastroenterol,* 1997, vol. 92, 706-7
- **Jonsson et al.** *FEMS Microbiol.lett.,* 2001, vol. 204, 19-22
- **Conly JM ; Stein K.** *Am J Gastroenterol.,* March 1992, vol. 87 (3), 311
- **Conly JM ; Stein K.** Quantitative and qualitative measurements of K vitamins. *Am J Gastroenterol.,* March 1992, vol. 87 (3), 311